# EUROPEAN PATENT SPECIFICATION

(11) **EP 1 159 960 B1**
(45) Date of publication and mention of the grant of the patent: **18.12.2002**
(21) Application number: 01119117.8
(22) Date of filing: 15.05.1997
(51) Int. Cl.: A61K 31/196, A61K 9/20, A61K 47/02, A61P 19/02

(54) **Pharmaceutical compositions based on diclofenac**
Arzneimittel bestehend aus Diclofenac
Compositions pharmaceutiques à base de diclofénac

(30) Priority: 17.05.1996 IT MI960992
(43) Date of publication of application: 05.12.2001
(62) Divisional of application: 97927045.1
(73) Proprietor: APR APPLIED PHARMA RESEARCH S.A., 6828 Balerna (CH)
(72) Inventor: Reiner, Alberto, 22100 Como (IT); Reiner, Giorgio, 22100 Como (IT)
(74) Representative: Pistolesi, Roberto

(56) References cited:
- EP-A- 0 236 855
- EP-A- 0 466 650
- EP-A- 0 523 376
- EP-A- 0 642 784
- WO-A-95/32737
- WO-A-96/14839
- WO-A-97/02017

## Description

The present invention relates to new pharmaceutical compositions containing the sodium or potassium salt of [(2,6-dichloro-anilino)-2-phenyl]-2-acetic acid (more commonly known as diclofenac) in combination with sodium or potassium bicarbonates or mixtures thereof.

Diclofenac is a non-steroidal drug which is widely dispensed and used owing to its well-known analgesic, anti-pyretic, anti-arthritic, anti-phlogistic and anti-rheumatic properties; its structural formula is indicated below.

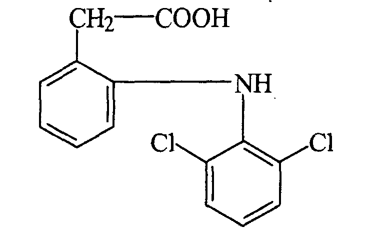

Diclofenac is generally taken orally in the form of normal tablets or tablets covered with coatings resistant to gastric juices, or rectally, or by injection, or topically.

The possibility of taking it in the form of sweets, tablets dissolving in the mouth, drages, chewing gum or other similar pharmaceutical forms or in formulations for the extemporary preparation of diclofenac-based aqueous solutions and/or suspensions would represent a different mode of administration which is definitely more suitable, especially for children and elderly persons.

Owing to its poor solubility in water, diclofenac is normally used in salt form; the salts of diclofenac customarily used are those of sodium, potassium or other alkali and alkaline earth metals, together with salts of organic nature, such as the salts of basic amino acids, such as lysine, arginine and ornithine, or other pharmacologically acceptable organic bases which have the ability to render the resulting salt soluble in water.

The pharmaceutical compositions of the diclofenac salts for oral use are generally accompanied by side effects of not inconsiderable consequence. Diclofenac salts are characterised by a particularly unpleasant and bitter taste and by the fact that they produce a sensation of strong astringency and cause an especially intense form of irritation in the buccal cavity, especially in the area of the larynx.

Although the first problem has been partly solved by using flavourings which are able in some manner to mask the taste, satisfactory solutions have still not been proposed for the two remaining problems.

Therefore, the pharmaceutical compositions containing diclofenac salts still have a poor palatability which limits their adoption and possible fields of application, despite the excellent therapeutic effect with which they are associated.

The subject of the present invention is that it has now surprisingly been found that, by adding sodium or potassium bicarbonates or mixtures thereof to the diclofenac salts, together with customary excipients and adjuvants, pharmaceutical compositions can be obtained which are for the most part free from the side effects mentioned above and especially from the astringent sensation in the area of the larynx.

The amount of such bicarbonates added to the compositions containing diclofenac is from 20 to 80 % by weight, based on the weight of the acid-form diclofenac.

It has also been found, and forms a second subject of the present invention, that the addition of flavouring substances selected from mint, aniseed, ammonium glycyrrhizinate and mixtures thereof to the compositions containing the diclofenac salts and sodium or potassium bicarbonates produces a synergistic effect which completely eliminates all the above-mentioned side effects, providing pharmaceutical compositions which are entirely palatable (and/or drinkable in the case of those used for the preparation of solutions and/or suspensions) and free from aftertaste.

It has now surprisingly been found that the use of sodium or potassium bicarbonates for the preparation of the compositions of the present invention permit constant, reproducible and foreseeable blood levels of the active ingredient, as it will be clear from example 4, with the consequent indisputable advantages from the therapeutic point of view.

Finally, it has also surprisingly been found that the combined use of sodium or potassium bicarbonates and the above-mentioned flavouring substances yields diclofenac-based pharmaceutical compositions in which the active ingredient is released more rapidly compared with normal formulations, bringing about higher blood levels and therefore a more immediate therapeutic effect.

The flavouring substances may be used as such or supported on inert materials, for example maltodextrin, in order to obtain a better distribution of the granulates and to facilitate excellent dispersibility of the flavouring in solution. Preferably, they are absorbed on maltodextrin with a power of 1 to 2000 and 1 to 1000.The amount of flavouring substances in its pure form is also preferably from 1/5 to 3 times the weight of the acid-form diclofenac. These flavouring substances are used in the implementation of the present invention without altering their organoleptic properties and without depriving them of their intrinsic qualities of flavourings which are liposoluble and generally oily in the pure state.The following Examples are given purely by way of illustration.

### Example 1 - Composition dissolving instantly in water

| **Active ingredients** | | |
|---|---|---|
| 1) | Diclofenac potassium salt* | 50 mg |
| 2) | Potassium bicarbonate | 22 mg |
| 3) | Mint flavouring on maltodextrin(1:2000)** | 60 mg |
| 4) | Aniseed flavouring on maltodextrin (1:1000)*** | 104 mg |

| - Excipients and adjuvants | | |
|---|---|---|
| 5) | Saccharin | 4 mg |
| 6) | Aspartame | 10 mg |
| 7) | Mannitol | 50 mg |
| 8) | Saccharose*** *q.s. | 2 g |

| | | |
|---|---|---|
| *If it is desired to prepare compositions based on diclofenac sodium salt, it is advantageous to use sodium bicarbonate in a quantity of approximately 38% by weight based on the weight of the diclofenac sodium salt present.Sodium carbonate may also be added to the sodium bicarbonate, maintaining the following optimum proportions: 27 % of sodium bicarbonate and 4-5 % of sodium carbonate, always based on the amount by weight of diclofenac sodium salt present. | | |
| ** The title of the pure mint essence, as obtained according to the Dean-Stark method, is of 18% by weight; the related amount is therefore in this case of 10.8 mg. | | |
| *** The title of the pure anise essence, as obtained according to the Dean-Stark method, is of 14.5% by weight, the related amount is therefore in this case of 16 mg. | | |
| **** The presence of saccharose is not strictly necessary; in its absence, a composition having a very limited granulate content is obtained which is perfectly soluble in contact with water. In that case, nothing is changed from the point of view of tolerability in contact with the mucosa and from the point of view of the palatability of the drinkable solution. | | |

### - Preparation

Components 1, 2, 5, 6 and 7 are mixed in a suitable mixer, and the mixture so obtained is wetted with 95% ethanol. Granulation is carried out with a 66 mm mesh and the granulate is preferably dried in a current of air.

Components 3, 4 and 8, which have already been granulated using a mesh of the same granulometry, are then added and the whole is mixed.

The mixture is then introduced into a metering machine filling packets or similar containers.

### Example 2 - Tablet for dissolving in the mouth

| - Active ingredients | | |
|---|---|---|
| 1) | Diclofenac potassium salt* | 50 mg |
| 2) | Potassium bicarbonate | 35 mg |
| 3) | Mint flavouring on maltodextrin**(1:2000) + gum arabic (E 414) | 50 mg |
| 4) | Aniseed flavouring (1:1000) on maltodextrin*** + silicon dioxide (E 551) | 120 mg |

| - Excipients and adjuvants | | |
|---|---|---|
| 5) | Saccharin | 50 mg |
| 6) | Aspartame | 12 mg |
| 7) | Mannitol | 20 mg |
| 8) | Saccharose **** | 300 mg |

| | | |
|---|---|---|
| *If it is desired to prepare compositions based on diclofenac sodium salt, it is advantageous to use sodium bicarbonate in a quantity of approximately 38% by weight based on the weight of the diclofenac sodium salt present.Sodium carbonate may also be added to the sodium bicarbonate, maintaining the following optimum proportions: 27 % of sodium bicarbonate and 4-5 % of sodium carbonate, always based on the amount by weight of diclofenac sodium salt present. | | |
| ** The title of the pure mint essence, as obtained according to the Dean-Stark method, is of 18% by weight; the related amount is therefore in this case of 10.8 mg. | | |
| *** The title of the pure anise essence, as obtained according to the Dean-Stark method, is of 14.5% by weight, the related amount is therefore in this case of 16 mg. | | |
| **** The presence of saccharose is not strictly necessary; in its absence, a composition having a very limited granulate content is obtained which is perfectly soluble in contact with water. In that case, nothing is changed from the point of view of tolerability in contact with the mucosa and from the point of view of the palatability of the drinkable solution. | | |

### Example 3 - Gum tablet

| - Active ingredients | | |
|---|---|---|
| 1) | Diclofenac potassium salt* | 50 mg |
| 2) | Potassium bicarbonate | 35 mg |
| 3) | Mint flavouring on maltodextrin** | 30 mg |
| 4) | Aniseed flavouring on maltodextrin*** | 80 mg |

| - Excipients and adjuvantsù | | |
|---|---|---|
| 5) | Mannitol | 30 mg |
| 6) | Menthol | 0.010 mg |
| 7) | Gum base | 600 mg |
| 8) | Sorbitol | 700 mg |
| 9) | Saccharin | 3 mg |
| 10) | Hydroxypropylmethylcellulose | 33 mg |
| 11) | Colouring agent | 7 mg |

| | | |
|---|---|---|
| *If it is desired to prepare compositions based on diclofenac sodium salt, it is advantageous to use sodium bicarbonate in a quantity of approximately 38% by weight based on the weight of the diclofenac sodium salt present.Sodium carbonate may also be added to the sodium bicarbonate, maintaining the following optimum proportions: 27 % of sodium bicarbonate and 4-5 % of sodium carbonate, always based on the amount by weight of diclofenac sodium salt present. | | |
| ** The title of the pure mint essence, as obtained according to the Dean-Stark method, is of 18% by weight; the related amount is therefore in this case of 10.8 mg. | | |
| *** The title of the pure anise essence, as obtained according to the Dean-Stark method, is of 14.5% by weight, the related amount is therefore in this case of 16 mg. | | |

### Example 4 - Comparative test

The packaged composition containing 50 mg of diclofenac potassium of Example 1 (formulation C) was subjected to a pharmacokinetic test for comparison with a similar composition not containing alkali metal carbonates and bicarbonates (formulation B), and with a second composition in tablet form (formulation A) produced by Ciba-Geigy (Voltaren Rapid ®), also in this case not containing alkali metal carbonates and bicarbonates, both formulations A and B containing 50 mg of diclofenac potassium.

This comparative evaluation was carried out on the same 6 healthy volunteers in accordance with the experimental plan described hereinafter.

Experimental scheme: Single-dose study using three methods in randomised cross- over with a wash-out of three days.
- Sampling times: Oh (before administration), 5 min, 10 min, 30 min, 45 min, 1h, 1.5h, 2h, 3h, 4h, 6h, 8h, after each administration.
- Blood sample treatment: 8MI in heparinised test tubes, centrifugation for 15 min at 1500 rev/min, subdivided into two fractions and subsequently frozen at -20°C.
- Times: wash-out of two days between treatments.
- Determination method: HPLC, with internal standard, sensitivity 10 ng/ml.

### Analysis method

- Column: Nova Pak C18, 3.9x150 mm, 4 µm Waters S.p.A. - Vimodrone, Italy.
- Eluant: NaH2PO4 0.01 M + 0.1 % TEA, pH 3.0 (H3PO4)/acetonitrile, 60/40
- Flow: 1.2 ml/min- Detection: UV/275 nm- Temperature: 30°C
- Injection: 50 al
- Analysis time: 16 min.

**Sample preparation** 10 al of the internal standard methanolic solution, and flufenamic acid (corresponding to 1320 ng) are added to 1 ml of defrosted plasma in 10 ml glass test tubes. The tubes are agitated in a Vortex mixer for 1 minute. 0.5 ml of a 0.5N HCl/lN NaCl solution is added. The whole is agitated in a Vortex mixer for 1 minute. 6 ml of a 95/5 n-hexane/isopropanol solution are added.

The mixture is then agitated in the Vortex mixer for a further 15 minutes. Centrifugation is carried out at 3000 rev/min for 15 minutes and the organic phase is transferred to fresh 10 ml glass test tubes and evaporated to dryness in a centrifugal evaporator under vacuum at ambient temperature. The whole is taken up in 200 al of a 70/30 acetonitrile/water solution, and the precipitate is dissolved under ultrasound for 2 minutes.

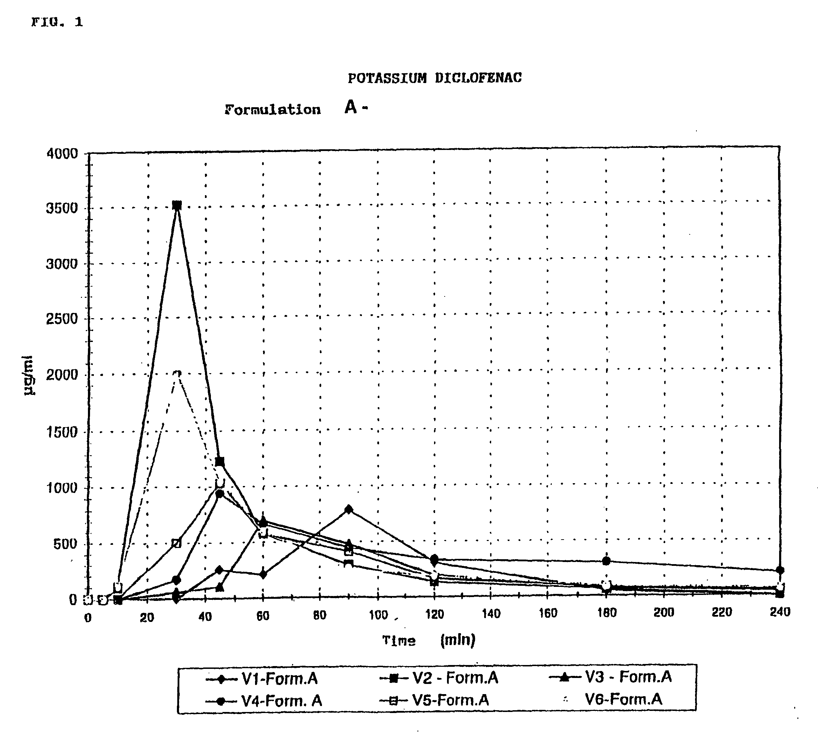

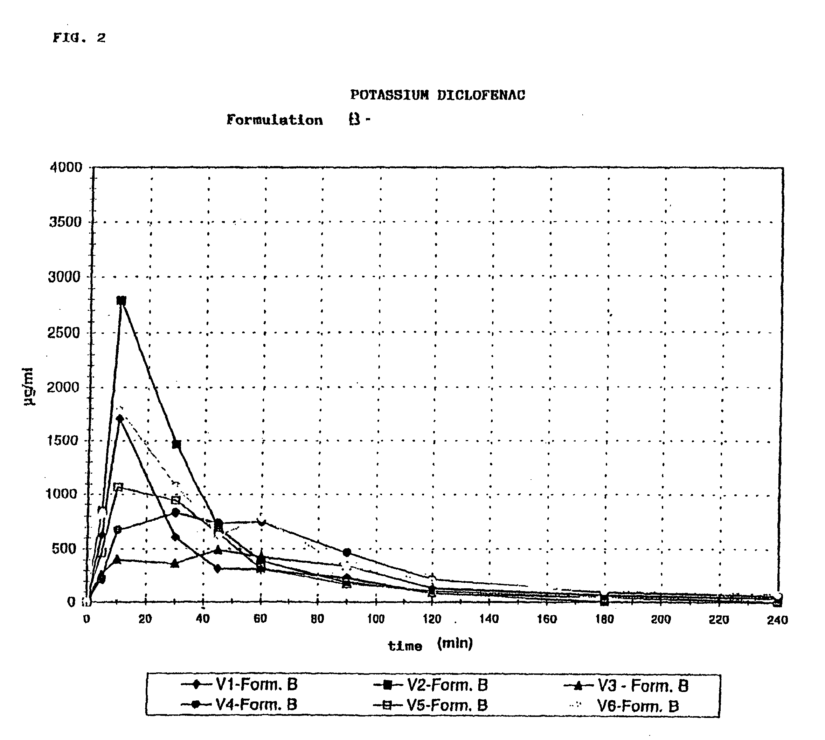

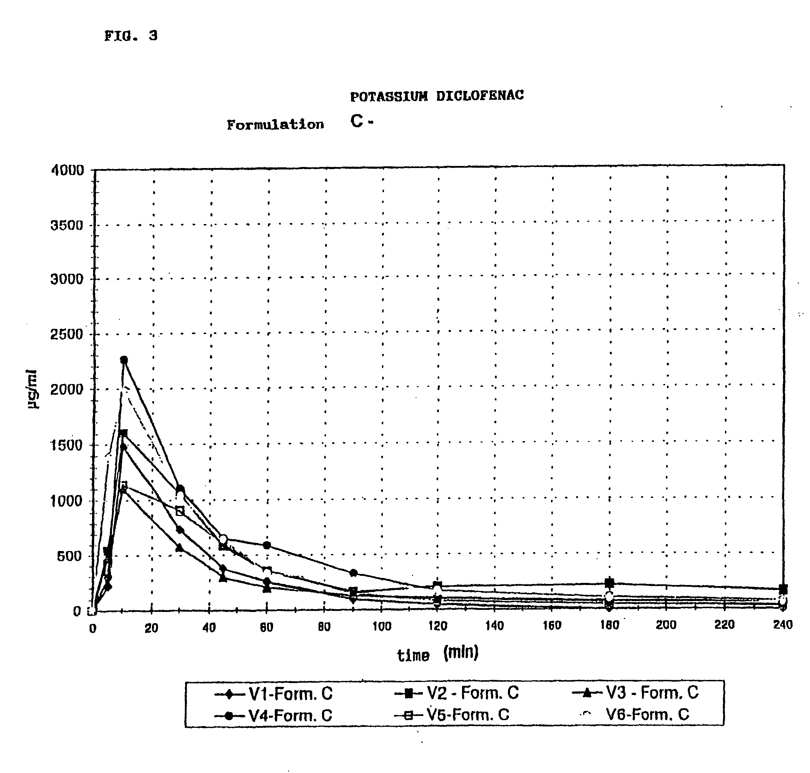

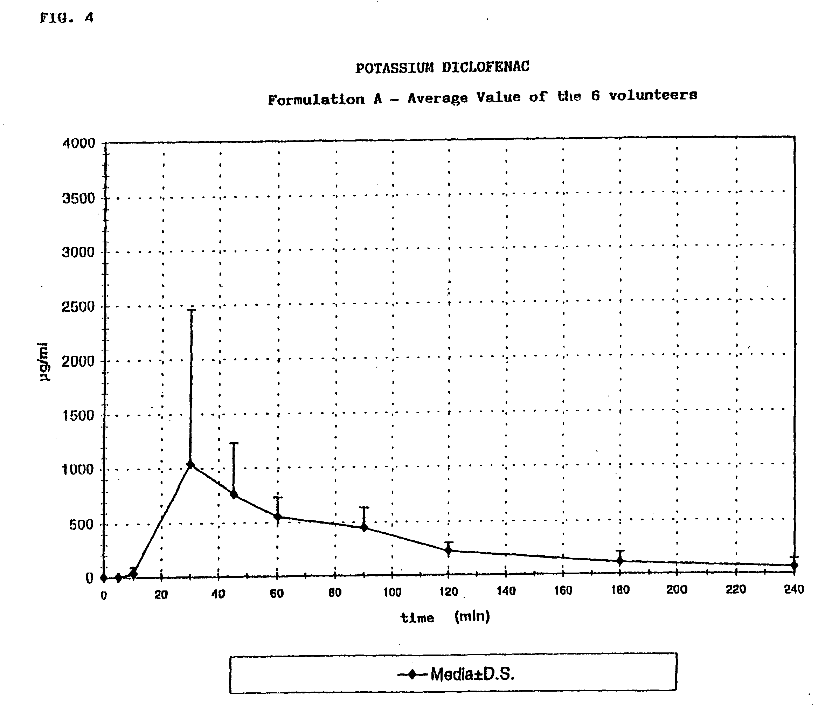

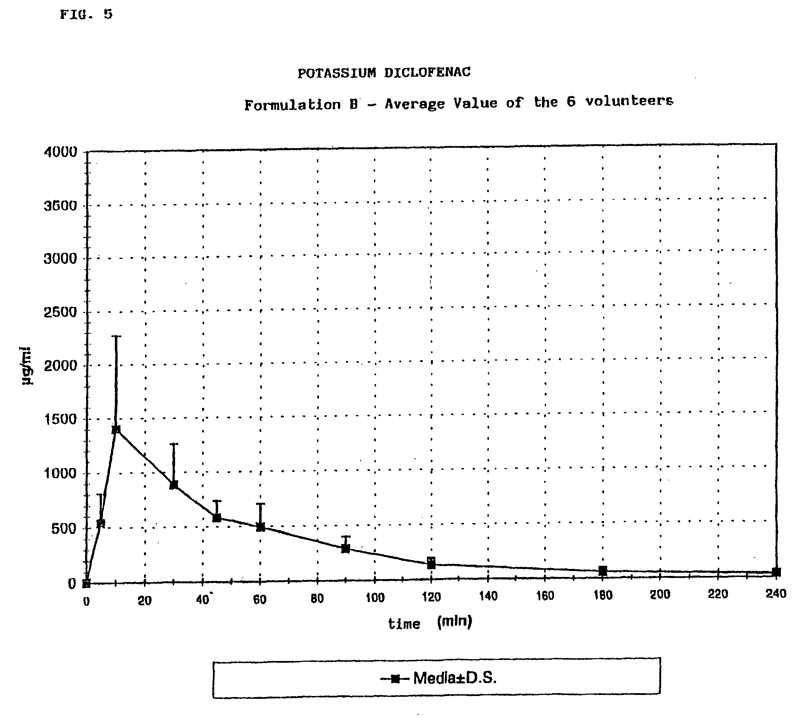

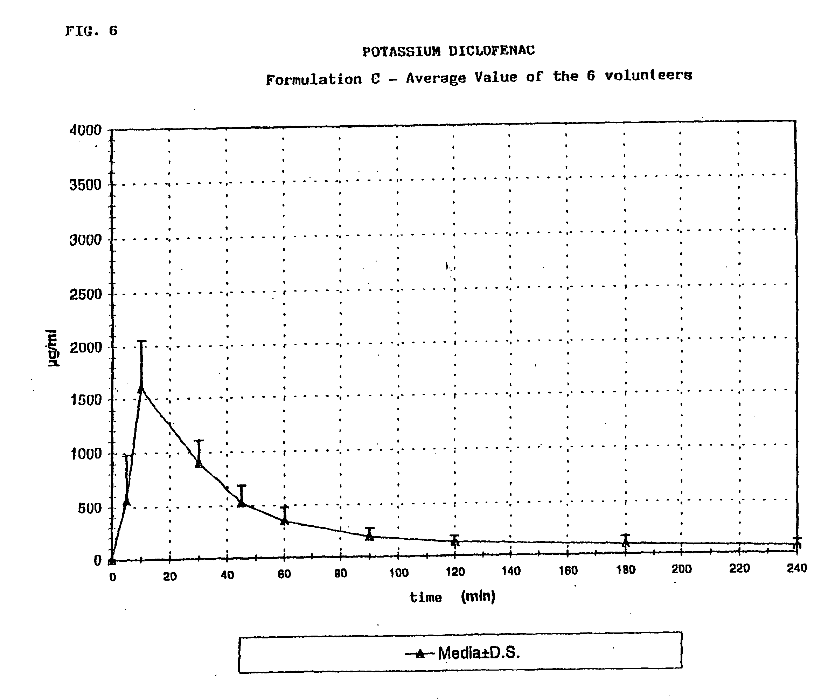

Figures 1, 2 and 3 show the concentrations of diclofenac in the blood of the six volunteers as regards formulations A, B (Ciba-Geigy comparative formulations) and C (formulation corresponding to the composition of Example 1), respectively.As will be appreciated, the blood concentration of the formulation of the present invention has, compared with the comparative formulations, a more constant and uniform pattern. This characteristic is also found in Figures 4, 5 and 6 which show the average values corresponding to the blood levels of the six volunteers together with the corresponding standard deviation.

The result is clear and surprising: compared with the sample compositions, the compositions of the present invention permit constant, reproducible and foreseeable blood levels of the active ingredient, irrespective of the characteristics of the volunteer (weight, age, etc), with the consequent indisputable advantages from the therapeutic point of view.

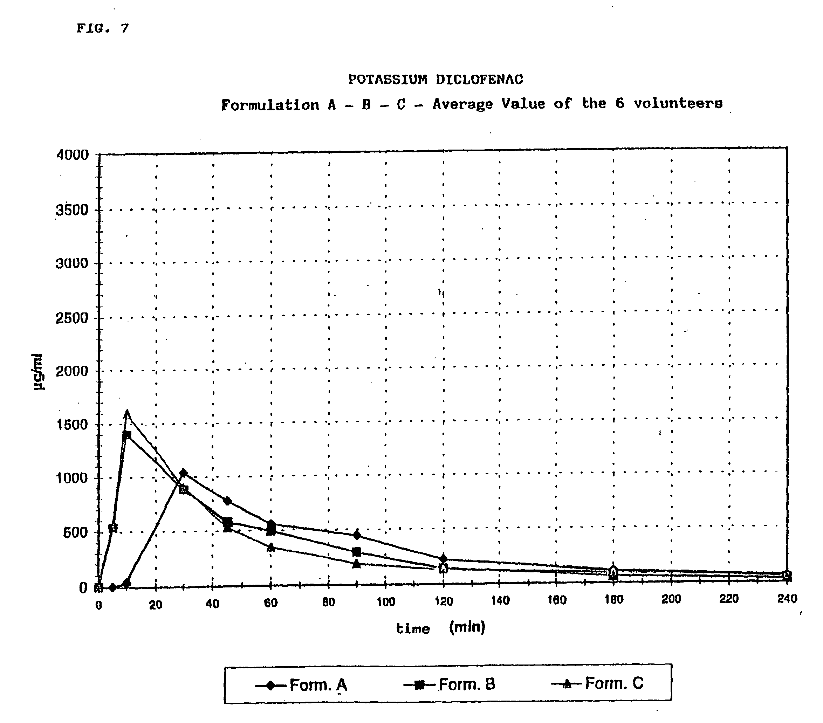

Finally, Figure 7 shows, by comparison, the graphs relating to the average values of the six volunteers (that is to say, the preceding Figures 4, 5 and 6); as will be noted, the formulation of the present invention permits, in addition to the advantages already mentioned, the attainment of a blood peak higher than that of the other formulations.

### Example 5 - Two layered tablet (fast and slow release)

| Fast release layer | | |
|---|---|---|
| 1) | Diclofenac potassium salt | 30 mg |
| 2) | Potassium bicarbonate | 30 mg |
| 3) | Lactose | 13.2 mg |
| 4) | Maize starch (intragranular) | 6 mg |
| 5) | Methyl cellulose | 0.12 mg |
| 6) | Sodium laurylsulfate | 0.06 mg |
| 7) | Maize starch (extragranular) | 9 mg |
| 8) | Crospovidone | 0.6 mg |
| 9) | Sodium carboxtmethylstarch | 1.5 mg |
| 10) | Magnesium stearate | 2.7 mg |
| 11) | Colloidal silicon dioxide | 0.6 mg |

| Slow release layer | | |
|---|---|---|
| 1) | Diclofenac potassium salt | 70 mg |
| 2) | Potassium bicarbonate | 30.8 mg |
| 3) | Lactose | 32.2 mg |
| 4) | Polyvinylpyrrolidone | 1.16 mg |
| 5) | Hydrpxypropylmethylcellulose | 70 mg |
| 6) | Magnesium stearate | 0.84 mg |
| 7) | Colloidal silicon dioxide | 0.21 mg |
| 8) | Talc | 3.92 mg |
| 9) | Polyethylene glycol | 0.56 mg |

### Example 6 - Drops

| | | |
|---|---|---|
| 1) | Diclofenac potassium salt | 75 g |
| 2) | Methyl p-oxybenzoate | 2.7 g |
| 3) | Propyl p-oxybenzoate | 0.3 g |
| 4) | Aspartame | 37.5 g |
| 5) | Potassium bicarbonate | 37.5 g |
| 6) | Glycerol | 300 g |
| 7) | Ethyl alcool | 450 g |
| 8) | Water q.s. | 1500 g |

Possible modifications:
a) Addition of sodium metabisulfite (0.06%)
b) Addition of sodium metabisulfite (0.06%)
   Mint flavouring (1.25%)
   Strawberry flavouring (0.75%)

### Example 7 - Drops

| | | |
|---|---|---|
| 1) | Diclofenac potassium salt | 37.5 g |
| 2) | Methyl p-oxybenzoate | 2.7 g |
| 3) | Propyl p-oxybenzoate | 0.3 g |
| 4) | Aspartame | 37.5 g |
| 5) | Potassium bicarbonate | 18.75 g |
| 6) | Saccharin | 6.0 g |
| 7) | Glycerol | 300 g |
| 8) | Ethyl alcool | 450 g |
| 9) | Water q.s. | 1500 g |

Possible modifications:
a) Addition of sodium metabisulfite (0.03%)
b) Addition of sodium metabisulfite (0.03%)
   Mint flavouring (1.25%)
   Strawberry flavouring (0.75%)

### Example 8 - Mouthwash

| | | |
|---|---|---|
| 1) | Diclofenac potassium salt | 0.75 g |
| 2) | Glycerol | 50 g |
| 3) | Sorbitol | 12 g |
| 4) | Saccharin | 0.5 g |
| 5) | Aspartame | 1.0 g |
| 6) | Methyl p-oxybenzoate | 0.5 g |
| 7) | Propyl p-oxybenzoate | 0.1 g |
| 8) | Mint flavouring | 1.0 g |
| 9) | Ethyl alcool | 100 g |
| 10) | Potassium bicarbonate | 0.33 g |
| 11) | Water q.s. | 500 ml |

### Example 9 - Gum-paste

| | | |
|---|---|---|
| 1) | Diclofenac potassium salt | 5.0 g |
| 2) | Glycerol | 630 g |
| 3) | Sodium benzoate | 5.0 g |
| 4) | Silica (Wessalon S® - Degussa) | 120 g |
| 5) | Silica (Siddent 9® - Degussa) | 80 g |
| 6) | Cellulose gum | 3.0 g |
| 7) | Polyethylenglycol 600 | 30 g |
| 8) | Sodium lauroyl sarcosinate (or sodium lauryl sulfate) | 60 g |
| 9) | Mint flavouring | 10 g |
| 10) | Sodium saccharin | 1.0 g |
| 11) | Aspartame | 3.0 g |
| 12) | Potassium bicarbonate | 2.2 g |
| 13) | Water q.s. | 1 kg |

### Example 10 - Tooth-paste

| | | |
|---|---|---|
| 1) | Diclofenac potassium salt | 5.0 g |
| 2) | Glycerol | 630 g |
| 3) | Sodium benzoate | 5.0 g |
| 4) | Silica (Wessalon S® - Degussa) | 20 g |
| 5) | Silica (Siddent 9® - Degussa) | 80 g |
| 6) | Cellulose gum | 3.0 g |
| 7) | Polyethylenglycol 600 | 30 g |
| 8) | Sodium lauroyl sarcosinate (or sodium lauryl sulfate) | 60 g |
| 9) | Mint flavouring | 10 g |
| 10) | Sodium saccharin | 1.0 g |
| | 11) Aspartame | 3.0 g |
| 12) | NaF | 1.0 g |
| 13) | Na₂FPO₃ | 4.0 g |
| 14) | Potassium bicarbonate | 2.2 g |
| 15) | Water q.s. | 1 kg |

### Example 11 - Tablet

| | | |
|---|---|---|
| 1) | Diclofenac potassium salt | 50 mg |
| 2) | Mannitol | 50 mg |
| 3) | Potassium bicarbonate | 22 mg |
| 4) | Maize starch (intragranular) | 10 mg |
| 5) | Methyl cellulose | 0.2 mg |
| 6) | Sodium laurylsulfate | 0.1 mg |
| 7) | Maize starch (extragranular) | 15 mg |
| 8) | Crospovidone | 1.0 mg |
| 9) | Sodium carboxymethylstarch | 2.5 mg |
| 10) | Magnesium stearate | 4.5 mg |
| 11) | Colloidal silicon dioxide | 10 mg |

## Claims

1. A pharmaceutical formulation for oral use containing diclofenac in sodium or potassium salt form together with sodium or potassium bicarbonates or mixtures thereof and customary excipients and adjuvants, **characterized in that** said sodium or potassium bicarbonates are present in an amount of from 20 to 80 % by weight based on the weight of diclofenac.

2. A pharmaceutical formulation according to claim 1 **characterized by** containing sodium bicarbonate and sodium diclofenac.

3. A pharmaceutical formulation according to claim 1 **characterized by** containing potassium bicarbonate and potassium diclofenac.

4. A pharmaceutical formulation according to claim 1 **characterized by** containing at least one flavouring substance selected from mint, aniseed and ammonium glycyrrhizinate.

5. A pharmaceutical formulation according to claim 4 **characterized in that** said at least one flavouring substance is present in pure form in an amount of from 1/5 to 3 times by weight based on the weight of diclofenac.

## Patentansprüche

1. Pharmazeutische Formulierung für die orale Verwendung, enthaltend Diclofenac in Natrium- oder Kaliumsalzform zusammen mit Natrium- oder Kaliumbicarbonaten oder Mischungen davon und üblichen Trägern und Hilfsmitteln, **dadurch gekennzeichnet, dass** die Natrium- oder Kaliumbicarbonate in einer Menge von 20 bis 80 Gew.%, basierend auf dem Gewicht des Diclofenacs, vorliegen.

2. Pharmazeutische Formulierung gemäss Anspruch 1, **dadurch gekennzeichnet, dass** sie Natriumbicarbonat und Natriumdiclofenac enthält.

3. Pharmazeutische Formulierung gemäss Anspruch 1, **dadurch gekennzeichnet, dass** sie Kaliumbicarbonat und Kaliumdiclofenac enthält.

4. Pharmazeutische Formulierung gemäss Anspruch 1, **dadurch gekennzeichnet, dass** sie mindestens eine Aromasubstanz enthält, ausgewählt aus Minze, Anis und Ammoniumglycyrrhizinat.

5. Pharmazeutische Formulierung gemäss Anspruch 4, **dadurch gekennzeichnet, dass** die mindestens eine Aromasubstanz in reiner Form in einer Menge von 1/5 bis 3 mal dem Gewicht, basierend auf dem Gewicht des Diclofenacs, vorliegt.

## Revendications

1. Composition pharmaceutique à usage oral contenant du diclofenac sous forme de sel de sodium ou de potassium de même que des bicarbonates de sodium ou de potassium ou des mélanges de ceux -ci ainsi que de excipients et adjuvants usuels, **caractérisée par le fait que** lesdits bicarbonates de sodium ou de potassium sont présents en une quantité de 20 à 80 % en poids basé sur le poids du diclofenac.

2. Composition pharmaceutique selon la revendication 1, **caractérisée par le fait qu'**elle contient du bicarbonate de sodium et du diclofenac de sodium.

3. Composition pharmaceutique selon la revendication 1, **caractérisée par le fait qu'**elle contient du bicarbonate de potassium et du diclofenac de potassium.

4. Composition pharmaceutique selon la revend ication 1, **caractérisée par le fait qu'**elle contient au moins une substance aromatisante choisie parmi la menthe, le glycyrrhizinate anisé et d'ammonium.

5. Composition pharmaceutique selon la revendication 4, **caractérisée par le fait que** ladite au moins une substance aromatique est présente sous forme pure en une quantité allant de 1/5 à 3 fois en poids basé sur le poids du diclofenac.
